# EUROPEAN PATENT APPLICATION

(11) **EP 4 617 344 A2**
(43) Date of publication of application: **17.09.2025**
(21) Application number: 25162631.3
(22) Date of filing: 10.03.2025
(51) Int. Cl.: C10L 1/02

(54) **JET REFERENCE FLUID FORMULATIONS FOR FUEL SURROGATES AND METHODS THEREOF**

(30) Priority: 13.03.2024 US 202463564762 P; 04.02.2025 US 202519045182
(71) Applicant: The Boeing Company, Arlington, VA 22202 (US)
(72) Inventor: BOZE, Jessica Adele, Arlington, 22202 (US); KOSILKIN, Ilya V., Arlington, 22202 (US); BELIERES, Jean-Philippe AJ, 22202, Arlington (US)
(74) Representative: Arnold & Siedsma

(57) **Abstract**

A jet reference fluid (JRF) composition and use in a method of testing is disclosed. The jet reference fluid includes a first hydrocarbon, and a second hydrocarbon, and where the first hydrocarbon and the second hydrocarbon may or may not include aromatic compounds. The jet reference fluid (JRF) composition may include one or more additives, such as glycol monomethyl ether. A method for testing compatibility of the jet reference fluid (JRF) with aviation fuel is also disclosed. The method can be used for evaluating one or more components or systems of an aerospace vehicle, including thermosets, thermoplastics, sealants, elastomers, metals, finishes, coatings, wiring or a combination thereof.

## Description

### CROSS-REFERENCE TO RELATED APPLICATIONS

This application claims the benefit of U.S. Provisional Patent Application No. 63/564,762, filed on March 13, 2024.

### TECHNICAL FIELD

The present teachings relate generally to evaluation of aerospace component exposure to fuels and, more particularly, to jet reference fluid formulations for testing of aerospace components for fuel exposure.

### BACKGROUND

Current systems and materials on airplanes were designed for conventional jet fuel compositions. There is an aviation industry goal to use more sustainable aviation fuel (SAF) and fuel sources, up to and including 100% sustainable aviation fuel (SAF). In order to move to cleaner and more efficient fuels, which may not have some of the chemical compounds of petroleum-based jet fuels, significant testing is necessary to ensure that systems and materials are compatible with proposed SAF formulations or compositions. This can include testing anything fuels contact on the airplane, such as primers, sealants, finishes, metals, composites, and O-rings, for example.

Sustainable aviation fuels (SAFs) are alternative fuels designed to reduce the environmental impact during their use in aviation by lowering greenhouse gas emissions as compared to conventional jet fuels. SAFs can be produced or refined from renewable resource ingredients, such as biomass, waste materials, or other carbon capture technologies. Additional flexibility can be garnered from blending SAFs with conventional jet fuels or replacing conventional jet fuels completely. The chemistry of jet fuel, including SAF, can have a range of composition and as a result a range of properties, and the resulting implications are two-fold: petroleum-derived jet fuel can have a range of composition, including 0% aromatics, and SAF or synthetic fuels can lack complete categories of chemistries which can be present in jet fuels, including aromatics and sulfur or nitrogen impurities, which can impact materials, they contact. An example can include mercaptans which can be found in crude oil, which can impact sealants in aerospace vehicles.

Therefore, it is desirable to have standards, materials, and methods available to better understand and categorize the compatibility of available SAFs with existing or proposed aviation components. It is important that the range of potential fuel compositions can be evaluated and that the boundary conditions for fuel composition as it relates to a material or system performance is evaluated.

### SUMMARY

The following presents a simplified summary in order to provide a basic understanding of some aspects of one or more embodiments of the present teachings. This summary is not an extensive overview, nor is it intended to identify key or critical elements of the present teachings, nor to delineate the scope of the disclosure. Rather, its primary purpose is merely to present one or more concepts in simplified form as a prelude to the detailed description presented later.

A jet reference fluid (JRF) composition is disclosed. The jet reference fluid includes a first hydrocarbon, and a second hydrocarbon, and where the first hydrocarbon and the second hydrocarbon may include no aromatic compounds. Implementations of the jet reference fluid (JRF) composition include where the first hydrocarbon may include iso-octane or the second hydrocarbon may include n-heptane. The first hydrocarbon can be present in an amount from about 1% by weight to about 20% by weight based on a total weight of the jet reference fluid. The second hydrocarbon can be present in an amount from about 50% by weight to about 90% by weight based on a total weight of the jet reference fluid. The jet reference fluid (JRF) composition may include glycol monomethyl ether (DiEGME). The glycol monomethyl ether (DiEGME) can be present in an amount from about 0.1% by weight to about 10% by weight based on a total weight of the jet reference fluid.

Another jet reference fluid (JRF) composition is disclosed, where the jet reference fluid includes a first hydrocarbon, and a second hydrocarbon, where one of the first hydrocarbon and the second hydrocarbon can include one or more aromatic compounds. Implementations of the jet reference fluid (JRF) composition can include where the first hydrocarbon may include toluene or the second hydrocarbon can include iso-octane. The jet reference fluid (JRF) composition can further include cyclohexane or n-heptane.

A method for testing compatibility with aviation fuel is disclosed. The method includes providing a quantity of a jet reference fluid (JRF), evaluating one or more components or systems of an aerospace vehicle. The method also includes exposing the quantity of jet reference fluid (JRF) to the one or more components or systems of an aerospace vehicle for a period of time. The method also includes evaluating the one or more components or systems of an aerospace vehicle after exposing the one or more components or systems of an aerospace vehicle to the jet reference fluid (JRF). Implementations of the method for testing compatibility with aviation fuel include where evaluating one or more components or systems of an aerospace vehicle can include weighing or visually inspecting the one or more components of an aerospace vehicle. Evaluating one or more components or systems of an aerospace vehicle after exposing the one or more components of an aerospace vehicle to the jet reference fluid (JRF) can include weighing or visually inspecting the one or more components or systems of an aerospace vehicle. The period of time may include a period of time from about 1 hour to about 72 hours, or from about 1hour to about 5,000 hours, or from about 24 hours to about 10,000 hours, in, for example, a long term soaking or exposure test for sealants or thermoplastics. The one or more components or systems of an aerospace vehicle may include a thermoset, a thermoplastic, a sealant, an elastomer, a metal, a finish, a coating, or a combination thereof. The one or more components or systems of an aerospace vehicle may include wiring. The one or more components or systems of an aerospace vehicle may include a fuel system. The jet reference fluid (JRF) may include iso-octane, n-heptane, toluene, or cyclohexane.

The features, functions, and advantages that have been discussed can be achieved independently in various implementations or can be combined in yet other implementations further details of which can be seen with reference to the following description.

### BRIEF DESCRIPTION OF THE DRAWINGS

The accompanying drawings, which are incorporated in and constitute a part of this specification, illustrate embodiments of the present teachings and together with the description, serve to explain the principles of the disclosure. In the figures:

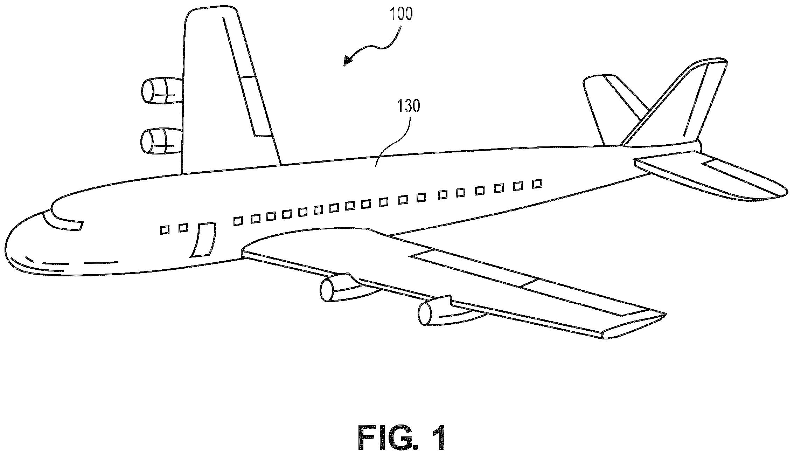

FIG. 1 depicts an application of an exemplary aerospace vehicle, in accordance with the present disclosure.

It should be noted that some details of the figures have been simplified and are drawn to facilitate understanding of the present teachings rather than to maintain strict structural accuracy, detail, and scale.

### DETAILED DESCRIPTION

Reference will now be made in detail to exemplary embodiments of the present teachings, examples of which are illustrated in the accompanying drawings. Wherever possible, the same reference numbers will be used throughout the drawings to refer to the same, similar, or like parts.

The present disclosure is directed to jet reference fluids (JRF) for sustainable aviation fuels that mimic the chemistry and behaviors of actual sustainable aviation fuels. The chemical makeup of sustainable aviation fuel can vary depending on the feedstock and refining process. The jet reference fluids of the present disclosure can be used to test and evaluate how sustainable aviation fuels interacts with airplane component materials or systems as compared to conventional petroleum-based jet fuel compositions.

The sustainable aviation fuel (SAF) jet reference fluids (JRF) mimic the boundary conditions for the chemistry of SAF, which would have the most significant impact on a given material. The chemical makeup of SAF can vary depending on the feedstock and refining process. Therefore, JRFs were developed to test how different types of fuel would interact with airplane materials, such as how a 100% synthetic paraffinic kerosene (SPK) may differ from a typical petroleum-derived Jet A with aromatics. For the purposes of this disclosure, boundary conditions for a solvent refers to the behavior of a solvent during use, contact with, or exposure to one or more candidate or test components for use in aerospace applications where a solvent represents a worst case for being most likely to degrade or damage the one or more candidate or test components for a period of time. In examples, certain materials can experience increased swelling from aromatic molecules. In other examples, different materials can have portions of the formulation or ingredients which are more readily soluble in one of an aromatic vs non-aromatic JRF. Petroleum jet fuels, including SAF, can contain mainly paraffins, that may be n-paraffins, iso-paraffins, cyclo-paraffins or a mixture of thereof, and may or may not contain aromatics. Therefore, it is important to evaluate materials and fuel systems while covering the compositional changes of petroleum jet fuels and synthetic jet fuels, including SAF.

The present disclosure is further directed to jet reference fluids (JRF) that been developed to have chemistry representing the boundary or worst-case for possible fuel chemistry and its impact on materials in the following general areas: thermoset materials, thermoplastic materials, sealants, elastomers, metals, finishes and coatings, wiring. The knowledge of materials and types of fluids that are the worst-case for exposure is also required to correlate this understanding with fuel chemistry. The approach for different types of materials can be further assessed and result in subsequent changes to the jet reference fluid recipe. In examples, Jet A/A-1 can have a range of 0-25% volume aromatics. However, it is rare for Jet A to have low or no aromatics. On the other hand, the most commonly produced synthetic blending component today is hydrotreated esters and fatty acids (HEFA), which has a maximum allowed aromatic content of 0.5% mass. In terms of the bulk constituents present in jet fuels, the concentration of aromatics has the most significant impact on solubility, surface tension, density, net heat of combustion, and yield sooting index (YSI). Other properties such as viscosity and freezing point depends on not only the hydrocarbon classes present but also the structural isomers within the classes.

The choices of solvents are based on a variety of factors, including the n- to iso- paraffin ratio commonly present in both conventional and sustainable fuels, the safety of handling - for example, while benzene may be the best boundary-condition molecule to represent aromatics in fuel for material compatibility it is has significant EHS concerns for use, and as a result toluene is used - and the range observed in fuel composition or allowed by specification (for example, 0-25% vol aromatics). The composition can thus be further tailored for the application. For example, smaller molecules will most impact elastomers and sealants, which can more effectively penetrate the material than larger ones. Epoxy materials are more susceptible when water is present, and as such, tests with JRFs used with epoxies can contain water.

Industrial aviation and various partners on industrial aviation are focused on improving and increasing the utilization of sustainable aviation fuel, up to and including 100% SAF-capability by 2030. The liquids of the present disclosure, known as jet reference fluids (JRFs), can be used to understand how airplanes interact with 100% sustainable aviation fuel (SAF). Where applicable, a definition of a 100% SAF capability goal can include 100% synthetic paraffinic kerosene (SPK), which would have no aromatic composition. Additionally, previous known testing with materials and fuel did not address the full spectrum of potential fuel chemistry. As such, there may be gaps in the present understanding of material performance in fuel, including current petroleum-derived jet fuel.

In contrast to the fluids described in the present disclosure, current jet reference fluids suffer from one or more of the following issues. They can contain a significant concentration of aromatics (therefore quite dissimilar to 100% SPK), they can be challenging to fabricate and consequently cost-prohibitive to procure (i.e., AMS2629 jet reference fuel), and/or they can be inconsistent from batch-to-batch. Additionally, existing published jet reference fluids do not have a notable impact on metallic materials, and it is known by those skilled in the art and from investigation of service history of metallic components that corrosion can occur in fuel tanks due to the composition of the fuel and/or water that can be present inside a fuel tank.

FIG. 1 depicts an application of an exemplary aerospace vehicle, in accordance with the present disclosure. As shown, the aerospace vehicle 100 may include an airplane or aircraft. The body 130 of the aircraft or vehicle 100 may include a number of separate components or systems, each of which can be exposed to fuels, and can be required to withstand the exposure to these fuels while still maintaining optimum performance throughout the service life of the component or system. The vehicle 100 may also or instead include other types of aircrafts such as helicopters, unmanned aerial vehicles (UAVs), spacecrafts, marine crafts, or the like, which are not shown herein. In other implementations, the vehicle 100 may be or include a car, a boat, a train, or the like. In yet other implementations, the system and method described below may not be implemented in a vehicle, but used to test components or systems that comprise or are incorporated in the vehicle 100. Aircraft propulsion is typically powered by aviation fuel, or conventional jet fuel. Due to the required specific energy, or energy per unit mass, aviation fuels are distinct from other classes of fuels. The extended energy storage capacity of aviation fuel or kerosene fuel can enable, for example, the possibility of long-haul flights. There are numerous varieties of aviation gasoline, each with unique properties, applications, and requirements. The same can also apply to candidate or more recently developed sustainable aviation fuels (SAFs). In examples, fuel tank materials, such as coatings like organic paints or inorganic finishes, sealants, elastomers such as gaskets or O-rings, wiring, and other components such as fuel quantity indicating system (FQIS) which may include one or more of a densitometer, fuel pump, various engine components, heat exchangers, or combinations thereof. While an exemplary example is shown in an aviation or aerospace vehicle, other systems, such as but not limited to ground equipment fueling systems, hydro-mechanical units (HMUs), or other fueling infrastructures might be applicable as well.

Several exemplary JRF formulations include a non-aromatic JRF (N-JRF) comprised of 90% iso-octane and 10% n-heptane. The non-aromatic JRF compositions contain no aromatic compounds. An aromatic compound can be defined as a compound, included in a large class of unsaturated chemical compounds characterized by one or more planar rings of atoms joined by covalent bonds of two different kinds. The unique stability of these compounds is referred to as aromaticity. Examples of aromatic hydrocarbons or aromatic compounds that can be applicable for JRF compositions of the present disclosure include, but are not limited to heteroarenes, compounds where at least one methine or vinylene (-C= or -CH=CH-) group is replaced by a heteroatom such as oxygen, nitrogen, or sulfur; or compounds that are similar to or derivatives of benzene, including illustrative aromatic compounds such as toluene, ethyl benzene, xylene, mesitylene, phenylhexane, biphenyl, phenol, aniline, nitrobenzene, benzoic acid, and the like. As such, these aromatic compounds can include aromatic rings that are either substituted or unsubstituted. For example, the aromatic rings can be substituted with functional groups such as hydroxy, alkoxy, halogen, nitro, alkanes, such as heptylbenzene), and the like, or combinations thereof. One or more of these aforementioned heteroarenes, heteroatom aromatics, or otherwise substituted aromatic compounds may be present in smaller percentages of a total composition, for example, 50% or less, and may not be present in some examples. An exemplary high aromatic JRF (HA-JRF) can be comprised of 30% toluene, 50% iso-octane, 10% cyclohexane, and 10% n-heptane.

Jet reference fluids (JRF) of the present disclosure can enable fluid exposure testing with materials that behave as surrogates for boundary conditions of candidate SAFs. The use and methods herein can be incorporated in testing for compatibility of one or more components on an airplane or aerospace vehicle utilizing, but also provide a repeatable outlook for system compatibility as well. Testing with the JRF fluids of the present disclosure can further enable the understanding of the boundary conditions of these systems that are in contact with or may be in contact with SAFs. The JRFs described herein are fluids made from simple materials that are controllable and reproducible, providing a means to facilitate comparison of data, using simple formulations and test methods using repeatable fluids and methods. In systems where aggressive environmental outlooks or plans include becoming 100% SAF capable within several years, it is important that any materials or systems that the candidate SAF comes in contact with can be evaluated for compatibility.

While formulations may be known, the JRFs of the present disclosure are designed to test the extreme compositional boundaries of the actual SAFs proposed to be used in the system or in exposure to the component. Findings and methods can be shared and compared with similar industry partners as a standardized method for testing. When SAFs are produced, there can be a compositional distribution, however, the JRF are representative of isolates of molecules within the SAF composition. If the system or component can pass the test method using JRFs, it should be fairly obvious that issues with the actual SAF will be reduced or non-evident. Components of aerospace vehicles can include groups of materials such as sealants, thermoplastics, thermosets, finishes, materials, and relevant fuel system and/or components. An additional advantage is that the manufacturer or provider of a specific SAF is immaterial, as the use of the JRFs and the method described herein are not limited to evaluation of exposure with any specific fuel.

Compatibility testing and additional testing methods can help ensure that SAFs can be safely used in existing aircraft engines without causing any unforeseen operational issues. Existing examples of compatibility testing include material compatibility testing, engine performance testing, fuel system testing, storage and handling tests, additive compatibility testing, or cold flow properties testing, among others. Material compatibility testing can include evaluating the compatibility of materials used in fuel systems such as seals, hoses, gaskets, and other materials with exposure to SAFs, thus understanding or ensuring that SAFs do not degrade or corrode materials over time. Engine performance testing can assess the impact of SAFs on engine performance, including efficiency, thrust, and emissions. The verification that SAFs meet the required specifications for combustion in aviation engines can be another companion test as well. Fuel system testing can examine the behavior of SAFs in fuel systems, while considering factors like flow characteristics and filtration, and ensuring that SAFs do not clog or damage fuel system components and atomize sufficiently within the combustion chamber. Storage and handling tests project and examine the effects of long-term storage on SAF properties, thus providing an outlook on handling procedures for SAFs and confirming that these handling or storage procedures are similar to conventional jet fuels. Cold flow properties testing can assess the performance of SAFs under colder conditions, such as low temperatures at high altitudes to avoid issues such fuel freezing or component precipitation. Additional tested or evaluated responses to JRF exposure can include system response to fuel composition such as a fuel quantity indicating system (FQIS), which can utilize either capacitance or ultrasonic information in which properties like dielectric constant vs. density and bulk modulus are important indicators of performance. Additional physical property testing such as tensile strength, elongation, modulus, and others for elastomers can be used, as well as adhesion for paints, or corrosion testing for metals.

Examples of sustainable aviation fuel (SAF) compositions or manufacturing methods can include hydroprocessed esters and fatty acids (HEFA), synthesized iso-paraffin from hydroprocessed fermented sugars (SIP), alcohol-to-jet (ATJ), hydroprocessed lignocellulosic biomass (HLB), and the like. Illustrative examples of hydroprocessed esters and fatty acids (HEFA) include materials derived from vegetable oils or animal fats, which can be chemically similar to traditional jet fuels, that also include a low sulfur content. Synthesized iso-paraffin from hydroprocessed fermented sugars (SIP) can be produced through the fermentation of sugars, followed by hydroprocessing, and they offer a high energy density similar to that of conventional aviation fuel. Examples of alcohol-to-jet (ATJ) made fuels can be derived from alcohols like ethanol or butanol, which are converted into jet fuel through a series of additional chemical processes. Hydroprocessed Lignocellulosic Biomass (HLB) fuels can be obtained from non-edible plant materials like wood, grasses, or agricultural residues with further hydroprocessing of lignocellulosic biomass. While these are some example pathways for making synthetic blending components or fuels, feedstocks, and end compositions, these categories are interconnected to some extent and can overlap. HEFA, SIP, ATJ are all examples of pathways to make fuel. HLB is a specific category of feedstock that can be utilized by different pathways depending on how it is processed. Fuel compositions include 100% synthetic paraffinic kerosene (SPK), which can be made by the HEFA, SIP, and ATJ pathways. However, it should be noted that the composition of the SPK can vary based on the feedstock and pathway, including conditions within a particular pathway such as, for example, temperature, catalyst, catalyst life, and fractionation. Other main categories of synthetic blending components contain aromatics and, in development, include a 100% cycloparaffinic fuel (CPK) and 100% synthetic aromatic kerosene (SAK). While SAK will not likely be used as a neat fuel, it can be used as a synthetic blending component. Examples of the former are synthesized paraffinic kerosene plus aromatics (Annex 5), and ATJ-SKA (ASTM D7566 Annex 8). There are also opportunities to blend these synthetic blending components together to make a fully synthetic "drop-in" fuel which falls within the petroleum-derived Jet A experience (e.g., blend of HEFA and SAK).

In examples of a method for testing compatibility with aviation fuel, several steps can be implemented, including providing a quantity of a jet reference fluid (JRF), evaluating one or more components or systems of an aerospace vehicle, exposing the quantity of jet reference fluid (JRF) to the one or more components or systems of an aerospace vehicle for a period of time, and evaluating the one or more components or systems of an aerospace vehicle after exposing the one or more components or systems of an aerospace vehicle to the jet reference fluid (JRF). The method for testing compatibility with aviation fuel can include evaluating one or more components or systems of an aerospace vehicle by weighing or visually inspecting the one or more components of an aerospace vehicle. The method for testing compatibility with aviation fuel can include evaluating one or more components or systems of an aerospace vehicle after exposing the one or more components of an aerospace vehicle to the jet reference fluid (JRF) which includes weighing or visually inspecting the one or more components or systems of an aerospace vehicle. In examples, the period of time can be a period of time from about 1 hour to about 10,000 hours. In other examples, the one or more components or systems of an aerospace vehicle can include a thermoset, a thermoplastic, a sealant, an elastomer, a metal, a finish, a coating, or a combination thereof. In alternate examples, the one or more components or systems of an aerospace vehicle includes wiring, which can incorporate one or more conductive metals or insulating or sheathing materials. The one or more components or systems of an aerospace vehicle can include a fuel system. The jet reference fluid (JRF) used in the method can include any of the JRFs described within the present disclosure. It should be noted that the fluid exposure parameters, including time and temperature can vary based on the material, as will the evaluation of that material after fluid exposure. For example, test exposure conditions can include times up to 10,000 hours, which can be referred to as a "long term soak" and temperatures from room temperature, which is considered to be approximately 25°C up to temperatures of about 75°C. Portions of the testing protocol can include, for certain materials, can include "switch-loading" where the sample exposure is switched between a high and low aromatic content jet reference fluid over time. It is known from prior testing of nitrile butadiene rubber (NBR) O-rings that this can impact the performance of the material differently as compared to only using a single test fluid. With NBR, jet fuel aromatic compounds can displace the plasticizers within the material. Then, when the testing is switched to a fuel without aromatics the aromatic compounds that were absorbed into the material partition out into the fuel and the O-ring shrinks in volume, which can result in leaks. A similar effect of extracting discrete components of a formulation of a materials can occur during switch-loading and it should be known if such a phenomenon can degrade the performance of a material over time.

One or more jet reference fluid (JRF) compositions of the present disclosure can include a first hydrocarbon and a second hydrocarbon, wherein the first hydrocarbon and the second hydrocarbon comprise no aromatic compounds. The first hydrocarbon, for example, can be or include iso-octane. The second hydrocarbon, for example, can include n-heptane. These hydrocarbons can also include cyclohexane, heptane, or any of the hydrocarbons described herein. Examples of the jet reference fluid (JRF) compositions include where the first hydrocarbon is present in an amount from about 1% by weight to about 20% by weight based on a total weight of the jet reference fluid, or where the second hydrocarbon is present in an amount from about 50% by weight to about 90% by weight based on a total weight of the jet reference fluid. Either the first or second hydrocarbon fluid, or any additional fluids added therein, can be present in an amount from about 1% to about 99% by weight, or from 10% to about 90% by weight, or from 20% to about 80% by weight, or from 30% to about 70% by weight, or from 40% to about 60% by weight, or from 50% to about 75% by weight. In examples, the jet reference fluid (JRF) composition can also include one or more additives, such as, for example, glycol monomethyl ether (DiEGME). This or other additives can be present in an amount from about 0.1% by weight to about 10% by weight, or from 1% by weight to about 20% by weight, or from 2% by weight to about 5% by weight based on a total weight of the jet reference fluid. In examples, the jet reference fluid (JRF) composition includes where one of the first hydrocarbon and the second hydrocarbon include one or more aromatic compounds, such as toluene.

Examples shown below are exemplary compositions of jet reference fluid mixtures:

**Table 1: Exemplary Non-aromatic Jet Reference Fluid (N-JRF) composition**

| **Constituent** | **Volume %** |
|---|---|
| Iso-octane | 90± 1 |
| n-heptane | 10± 1 |

**Table 2: Exemplary Aromatic Jet Reference Fluid composition with glycol monomethyl ether additive (JRF-DiEGME)**

| **Constituents FL1** | **Volume %** |
|---|---|
| Toluene | 30 ± 0.5 |
| Cyclohexane | 10 ± 0.5 |
| Iso-octane | 50 ± 0.5 |
| n-heptane | 10 ± 0.5 |
| DIEGME | 0.6 FL1 |
| **FL1** By weight percent of the total of the other four components | |
| Water 1.2% by volume of the total JRF mixture - i.e, 3.6 mL per 300 mL of test fluid | |

**Table 3: Exemplary Non-Aromatic Jet Reference composition with glycol monomethyl ether additive (N-JRF-DiEGME)**

| **Constituents FL1** | **Volume %** |
|---|---|
| Iso-octane | 90 ± 0.5 |
| n-heptane | 10 ± 0.5 |
| DIEGME | 0.6 FL1 |
| **FL1** By weight percent of the total of the other two components | |
| Water 1.2% by volume of the total JRF mixture - i.e, 3.6 mL per 300 mL of test fluid | |

**Table 4: Exemplary Aromatic Jet Reference Fluid composition with glycol monomethyl ether additive (JRF-LT)**

| **Constituents FL1** | **Volume %** |
|---|---|
| Toluene | 30 ± 0.5 |
| Cyclohexane | 10 ± 0.5 |
| Iso-octane | 50 ± 0.5 |
| n-heptane | 10 ± 0.5 |

The jet reference fluids listed in the table represent the various applicable boundary cases for fuel chemistry to assess material compatibility. These jet reference fluid recipes are intentionally designed to contain materials that can be easily procured. Therefore, the usage of these fluids should be repeatable and scalable. These materials are compatible with different fuel types. Testing can be simplified and expensive, and variable fluids such as, for example, AMS2629, which requires extensive laboratory testing to generate and is based upon Jet A which can also vary in composition batch to batch. The shelf life for AMS2629 can be limited and any extension of shelf life requires additional testing which is not readily available to most laboratories. With test fluid compositions of the present disclosure, the AMS2629 and other such materials will not be needed, resulting in cost savings over the testing required in various materials specifications. There can potentially be a significant impact on military aircraft, demonstrating that the materials used in the aircraft fuel tank (particularly primer) are compatible with DiEGME since this is an additive required for military fuel specifications. It should be noted that while DiEGME is required on military aircraft it is allowed in commercial fuel as well. If an operator decides that there is an advantage to DiEGME (e.g., biostatic properties) it may be used more frequently in the commercial fuel space.

Additional test fluids for evaluating metals and inorganic finishes exposure can include naphthenic acids and sulfur species as they are known impurities in crude oil which can be found in finished jet fuel and are known to impact metal corrosion. Such an example is a "synthetic sump" material as shown in Tables 5, 6, and 7 below.

**Table 5: "Synthetic Sump" recipe formulation**

| **Salt:** | **Weight Fraction** |
|---|---|
| CaCl₂ | 0.0050 |
| CdCl₂ | 0.1000 |
| MgCl₂ | 0.0050 |
| NaCl | 0.0100 |
| ZnCl₂ | 0.0010 |
| CrCl₃*6H₂O | 0.0001 |
| CuCl₃*2H₂O | 0.0001 |
| FeCl₃ | 0.0005 |
| MnCl₂*4H₂O | 0.0005 |
| NiCl₂*6H₂O | 0.0001 |
| PbCl₂ | 0.0001 |
| Deionized H₂O | (remaining) 0.8776 |

**Table 6: Sulfur species example materials**

| **Sulfur Species** | |
|---|---|
| Tert-Butyl Mercaptan | 30 mg/L |
| Butyl Disulfide | 495 mg/L |
| Benzene Sulfonic Acid | 990 mg/L |
| Ammonium Sulfate | 990 mg/L |

**Table 7: Napthenic acid example materials**

| **Naphthenic Acids** | |
|---|---|
| 1-Naphthoic Acid | 46 mg/L |
| Oleic Acid | 46 mg/L |

While the present teachings have been illustrated with respect to one or more implementations, alterations and/or modifications may be made to the illustrated examples without departing from the scope of the appended claims. For example, it may be appreciated that while the process is described as a series of acts or events, the present teachings are not limited by the ordering of such acts or events. Some acts may occur in different orders and/or concurrently with other acts or events apart from those described herein. Also, not all process stages may be required to implement a methodology in accordance with one or more aspects or embodiments of the present teachings. It may be appreciated that structural objects and/or processing stages may be added, or existing structural objects and/or processing stages may be removed or modified. Further, one or more of the acts depicted herein may be carried out in one or more separate acts and/or phases. Furthermore, to the extent that the terms "including," "includes," "having," "has," "with," or variants thereof are used in either the detailed description and the claims, such terms are intended to be inclusive in a manner similar to the term "comprising." The term "at least one of" is used to mean one or more of the listed items may be selected. Further, in the discussion and claims herein, the term "on" used with respect to two materials, one "on" the other, means at least some contact between the materials, while "over" means the materials are in proximity, but possibly with one or more additional intervening materials such that contact is possible but not required. Neither "on" nor "over" implies any directionality as used herein. The term "conformal" describes a coating material in which angles of the underlying material are preserved by the conformal material. The term "about" indicates that the value listed may be somewhat altered, as long as the alteration does not result in nonconformance of the process or structure to the illustrated embodiment. The terms "couple," "coupled," "connect," "connection," "connected," "in connection with," and "connecting" refer to "in direct connection with" or "in connection with via one or more intermediate elements or members." Finally, the terms "exemplary" or "illustrative" indicate the description is used as an example, rather than implying that it is an ideal. Other embodiments of the present teachings may be apparent to those skilled in the art from consideration of the specification and practice of the disclosure herein. It is intended that the specification and examples be considered as exemplary only, with a true scope of the present teachings being indicated by the following claims.

## Claims

1. A jet reference fluid (JRF) composition, comprising:
a first hydrocarbon; and
a second hydrocarbon; and
wherein the first hydrocarbon and the second hydrocarbon comprise no aromatic compounds.

2. The jet reference fluid (JRF) composition of claim 1, wherein the first hydrocarbon comprises iso-octane,
and/or
wherein the second hydrocarbon comprises n-heptane.

3. The jet reference fluid (JRF) composition of claim 1 or 2, wherein the first hydrocarbon is present in an amount from about 1% by weight to about 20% by weight based on a total weight of the jet reference fluid,
and/or
wherein the second hydrocarbon is present in an amount from about 50% by weight to about 90% by weight based on a total weight of the jet reference fluid.

4. The jet reference fluid (JRF) composition of any of the preceding claims, further comprising glycol monomethyl ether (DiEGME), preferably wherein the glycol monomethyl ether (DiEGME) is present in an amount from about 0.1% by weight to about 10% by weight based on a total weight of the jet reference fluid.

5. A jet reference fluid (JRF) composition, comprising:
a first hydrocarbon; and
a second hydrocarbon; and
wherein one of the first hydrocarbon and the second hydrocarbon comprise one or more aromatic compounds.

6. The jet reference fluid (JRF) composition of claim 5, wherein the first hydrocarbon comprises toluene, and/or wherein the second hydrocarbon comprises iso-octane.

7. The jet reference fluid (JRF) composition of claim 5 or 6, further comprising cyclohexane.

8. The jet reference fluid (JRF) composition of any of the claims 5-7, further comprising n-heptane.

9. A method for testing compatibility with aviation fuel, comprising:
providing a quantity of a jet reference fluid (JRF) according to any of the preceding claims,
evaluating one or more components or systems of an aerospace vehicle;
exposing the quantity of jet reference fluid (JRF) to the one or more components or systems of an aerospace vehicle for a period of time; and
evaluating the one or more components or systems of an aerospace vehicle after exposing the one or more components or systems of an aerospace vehicle to the jet reference fluid (JRF).

10. The method for testing compatibility with aviation fuel of claim 9, wherein evaluating one or more components or systems of an aerospace vehicle comprises weighing or visually inspecting the one or more components of an aerospace vehicle.

11. The method for testing compatibility with aviation fuel of claim 9 or 10, wherein evaluating one or more components or systems of an aerospace vehicle after exposing the one or more components of an aerospace vehicle to the jet reference fluid (JRF) comprises weighing or visually inspecting the one or more components or systems of an aerospace vehicle.

12. The method for testing compatibility with aviation fuel of any of the claims 9-11, wherein the period of time comprises a period of time of from about 1 hour to about 72 hours.

13. The method for testing compatibility with aviation fuel of any of the claims 9-12, wherein the one or more components or systems of an aerospace vehicle comprises one or more of the following:
a thermoset, a thermoplastic, a sealant, an elastomer, a metal, a finish, a coating, or a combination thereof, wiring;
a fuel system.

14. The method for testing compatibility with aviation fuel of any of the preceding claims 9-13, wherein the jet reference fluid (JRF) comprises iso-octane and n-heptane.

15. The method for testing compatibility with aviation fuel of any of the claims 9-13, wherein the jet reference fluid (JRF) comprises toluene and cyclohexane.
